# Europäisches Patentamt
# European Patent Office
# Office européen des brevets

(19)

(11) Veröffentlichungsnummer: **0 224 804**
**B1**

(12) **EUROPÄISCHE PATENTSCHRIFT**

(45) Veröffentlichungstag der Patentschrift:
24.05.89

(51) Int. Cl.⁴: **G01L 17/00**, A61B 3/16

(21) Anmeldenummer: 86116067.9

(22) Anmeldetag: 20.11.86

(54) Tonometer zur Ermittlung des Augeninnendruckes.

(30) Priorität: 26.11.85 CH 5047/85

(43) Veröffentlichungstag der Anmeldung:
10.06.87 Patentblatt 87/24

(45) Bekanntmachung des Hinweises auf die Patenterteilung:
24.05.89 Patentblatt 89/21

(84) Benannte Vertragsstaaten:
AT BE CH DE FR GB IT LI NL SE

(56) Entgegenhaltungen:
CH-A- 91 723
DE-C- 882 590
FR-A- 2 280 881
FR-A- 2 351 400
GB-A- 913 498
GB-A- 970 305
US-A- 3 805 595

PATENTS ABSTRACTS OF JAPAN, Band 6,
Nr. 46 (P-107)[924], 24. März 1982; &
JP-A-56 163 431 (SUMITOMO GOMU KOGYO
K.K.) 16-12-1981

(73) Patentinhaber: Interzeag AG, Rietbachstrasse 5,
CH-8952 Schlieren(CH)

(72) Erfinder: Trittenbass, Jean, Im Winkel 3,
CH-8600 Dübendorf(CH)

(74) Vertreter: Fillinger, Peter, Dr., Rütistrasse 1a,
CH-5400 Baden(CH)

## Beschreibung

Die Erfindung betrifft eine Vorrichtung gemäss dem Oberbegriff des Patentanspruchs 1.

Die Notwendigkeit, den Innendruck in einem Prüfkörper zu messen, ohne dass der Prüfkörper selbst angezapft oder angebohrt werden kann, ist vor allem aus der Augendiagnostik bekannt. Bei Glaukomen, einer durch einen ansteigenden Druck im Augeninnern charakterisierten Augenerkrankung ist es erforderlich, den Druck im Augeninnern, der beim gesunden Auge etwa 26,66 mbar (20 mm Hg) beträgt, zu bestimmen. Hierzu werden als Tonometer bezeichnete Messgeräte verwendet, die wegen ihres komplizierten Aufbaues von einem Arzt oder von einer hierzu speziell ausgebildeten Person bedient werden müssen.

Es ist auch ein Tonometer bekannt (Zeitschrift Arch Ophthalmol Bd. 101, Nov. 1983, S. 1791/1793), das der Patient selbst bedienen kann. Während bei der elektronischen Tonometrie bzw. -graphie, die von einem Arzt oder einer dazu ausgebildeten Person ausgeführt wird, die Kraft für die Applanation der Hornhaut gemessen wird, wird bei der selbst auszuführenden Tonometrie der Druck gemessen, der für das Einebnen der Hornhaut erforderlich ist. In beiden Fällen ist es jedoch erforderlich, die Hornhaut entweder durch ein Schmerzbetäubungsmittel oder durch eine dünne Kontaktlinse unempfindlich zu machen.

Hier setzt die Erfindung ein, der die Aufgabe zugrunde liegt, eine Vorrichtung zur Ermittlung des Innendruckes in einem Prüfkörper, die auch als Tonometer zur Bestimmung des Augeninnendruckes geeignet ist, so auszugestalten, dass die Messung durch die Prüfperson selbst problemlos durchgeführt werden kann. Insbesondere soll die Vorrichtung bei Verwendung als Tonometer die Bestimmung des Augeninnendruckes ermöglichen, ohne dass die Hornhaut vorpräpariert werden muss, d.h. dass die Messung sogar bei geschlossenem Augenlid durchgeführt werden kann.

Diese Aufgabe wird gemäss der Erfindung gelöst durch die kennzeichnenden Merkmale des Anspruches 1.

Die Erfindung ist aus der Zeichnung ersichtlich, in welcher mehrere Ausführungsbeispiele der Erfindung schematisch dargestellt und nachfolgend beschrieben sind. Es zeigen:

Fig. 1 eine schematische Darstellung eines Prüfkörpers und eines Messkörpers, bevor letzterer gegen den Prüfkörper gedrückt wird,

Fig. 2 eine schematische Darstellung eines Prüfkörpers und eines Messkörpers, nachdem gegen den Prüfkörper gedrückt wird,

Fig. 3 eine schematische Darstellung einer Vorrichtung zur Ermittlung des Innendruckes in einem Prüfkörper,

Fig. 4 eine schematische Darstellung einer weiteren Vorrichtung zur Ermittlung des Innendruckes in einem Prüfkörper,

Fig. 5 ein zweites Ausführungsbeispiel in gleicher Darstellung wie Fig. 3.

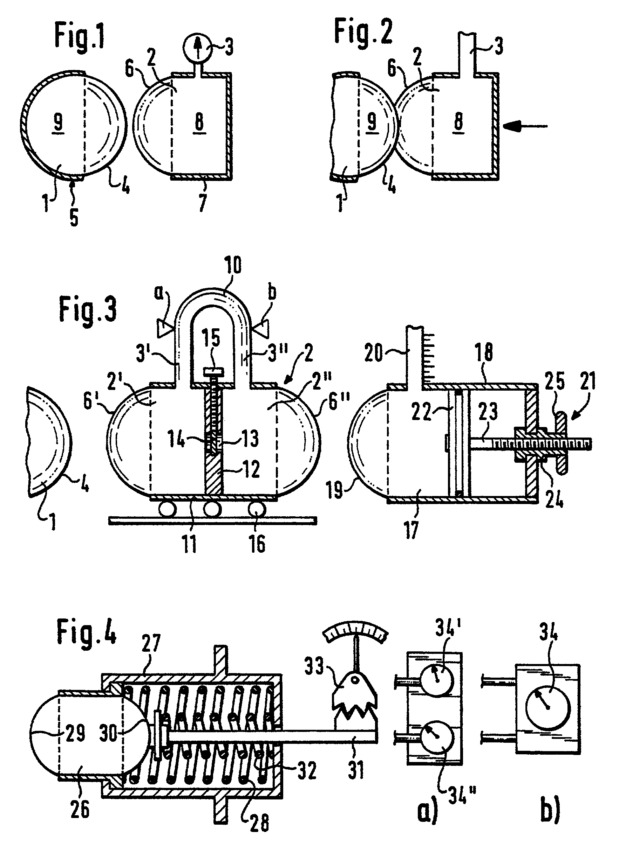

Fig. 1 zeigt diejenigen Mittel, die notwendig sind, um etwas über den Innendruck in einem Prüfkörper 1 aussagen zu können. Diese Mittel setzen sich aus einem Messkörper 2 und einem Messgerät 3 zusammen. Damit eine Aussage über den Innendruck des Prüfkörpers 1 gemacht werden kann, muss eine Wandpartie 4 der Wand 5 des Prüfkörpers 1 flexibel und elastisch verformbar sein. Auch der Messkörper 2 weist eine flexible Wandpartie 6 auf, während die übrige Wand 7 fest sein kann. Der Innenraum 8 des Messkörpers 2 kann mit einem Fluid, das heisst mit Luft, einem Gas oder einer Flüssigkeit gefüllt sein. Die Füllung des Prüfkörpers 1 kann beliebig sein. In seinem Innenraum herrscht ein bestimmter Überdruck gegenüber der Atmosphäre, der gemessen werden soll.

Das Messgerät 3 des Messkörpers dient dazu, den Druck im Innenraum 8 zu messen und anzuzeigen. In Fig. 1 ist das Messgerät 3 beispielsweise ein Zeigergerät. Ist die Füllung des Messkörpers eine Flüssigkeit, kann das Messgerät 3 ein Standrohr sein, wie dies in Fig. 2 dargestellt ist.

Wird nun der Messkörper 2 in Pfeilrichtung gegen den Prüfkörper 1 gedrückt, wird die flexible Wandpartie 6 des Messkörpers 2 eingedrückt, siehe Fig. 2. Da durch das Eindrücken der flexiblen Wandpartie 6 der Innenraum 8 des Messkörpers 2 kleiner wird, wird im Messgerät 3 ein entsprechend höherer Druck angezeigt. Ist ein Standrohr vorgesehen, wird ein entsprechend höheres Flüssigkeitsniveau angezeigt.

Ist aber umgekehrt der Innendruck im Innenraum 8 des Messkörpers 2 grösser als der Innendruck im Prüfkörper 1, wird die flexible Wandpartie 4 des Prüfkörpers 1 eingedrückt. Wird nun durch Entnahme von Flüssigkeit im Messkörper 2 der Innendruck solange gesenkt, bis die aneinander liegenden Wandpartien von Prüfkörper 1 und Messkörper 2 eben abgeflacht sind, stellt der Druck im Messkörper 2, bei dem dieser Zustand eintritt, den Druck im Prüfkörper 1 dar.

Nun ist diese Art der Feststellung des Innendruckes im Prüfkörper 1 recht umständlich, damit soll aber gezeigt werden, dass das Wesen der Erfindung darin besteht, den Druck im Innenraum eines Prüfkörpers 1 durch eine indirekte Druckmessung zu ermitteln. Im weitern ist es klar, dass der Druck, mit welchem der Messkörper 2 gegen den Prüfkörper 1 gedrückt wird, nur so gross ist, dass die elastische Verformung der flexiblen Wandpartie 4 des Messkörpers 2 gewährleistet ist.

Wird der Messkörper 2, siehe Fig. 3, mit zwei getrennten Messkammern 2', 2'' ausgerüstet, wird der Innendruck des Prüfkörpers 1 ebenfalls indirekt bestimmt. Die beiden Messkammern 2', 2'' weisen je ein Messgerät in Form von Standrohren 3', 3'' auf, die durch einen Verbindungsbogen 10 miteinander verbunden sind und in dem Luft eingeschlossen ist. Die Standrohre 3', 3'' mit dem Verbindungsbogen 10 können auch nach abwärts gerichtet sein. Ein solcher Hängerohrbogen benötigt anstelle einer Luftblase ein anderes Trennmedium, z.B. Quecksilber. Die Messgeräte können jedoch auch, wie bereits bei der Beschreibung von Fig. 1 und 2 erwähnt wurde, noch anders ausgebildet sein, z.B. als Zeigergerät oder dgl.

Der Messkörper 2 nach Fig. 3 besteht aus einem festen Gehäuse 11, z.B. einem Rohrabschnitt. Die beiden Messkammern 2', 2" sind durch eine feste Wand 12 voneinander getrennt, in der jedoch ein Durchgang 13 vorgesehen ist, der durch ein Abschlussorgan 14, z.B. einen Schieber, mittels eines Griffes 15 von aussen geöffnet und geschlossen werden kann.

Beide Messkammern 2', 2" weisen eine flexible Wandpartie 6', 6" auf. Das Gehäuse 11 des Messkörpers 2 ist möglichst reibungsfrei verschiebbar gelagert, was durch Rollen 16 dargestellt ist, auf denen das Gehäuse 11 lagert. Die Verschiebbarkeit kann jedoch auch in anderer Weise verwirklicht werden, z.B. dadurch, dass der Messkörper 2 pendelnd aufgehängt oder auf blattfederförmigen Gelenken abgestützt ist.

Mit dem Messkörper 2 wirkt ein weiterer Messkörper 17 zusammen. In seiner einfachsten Ausführung ist er als geschlossenes Gehäuse ausgebildet, dessen Wand 18 mindestens eine flexible, elastisch verformbare Wandpartie 19 aufweist.

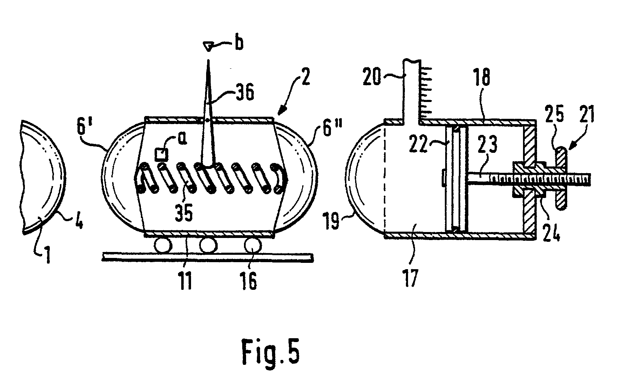

In Fig. 3 ist der weitere Messkörper 17 mit einem Messgerät 20 zur Messung des Innendruckes des weiteren Messkörpers 17 und mit einer Verstellvorrichtung 21 ausgerüstet. Die Verstellvorrichtung 21 setzt sich im wesentlichen aus einem Kolben 22 und einer Spindel 23 zusammen, welch letztere mit einer Spindelmutter 24 zusammenwirkt, die in der Wand 18 drehbar gelagert und durch einen Drehgriff 25 drehbar ist. Der Kolben 22 könnte durch eine flexible Wand ersetzt werden, auf die die Verstellvorrichtung 21 wirkt, siehe Fig. 4.

Zur Messung des Innendruckes in dem Prüfkörper 1 wird der Messkörper 2 auf seiner reibungsfreien Lagerung 16 mit Hilfe des weiteren Messkörpers 17 gegen den Prüfkörper 1 gedrückt, wobei je nach den bestehenden Innendrücken eine unterschiedliche Verformung der flexiblen Wandpartien auftreten kann. Es sei beispielsweise angenommen, dass der Innendruck des Prüfkörpers 1 grösser ist als die Innendrücke in den Messkammern 2', 2" und der Innendruck des weiteren Messkörpers 17. Zudem sei der letztere Innendruck kleiner als die Innendrücke in den Messkammern 2', 2". In diesem Fall werden die flexiblen Wandpartien 6' der Messkammer 2' und die Wandpartie 19 des weiteren Messkörpers 17 verformt. Im Messgerät 3' wird eine grössere Volumenverdrängung angezeigt als im Messgerät 3". Aus dieser Anzeige kann lediglich festgestellt werden, dass der Innendruck im Prüfkörper 1 grösser ist als die übrigen Innendrücke. Weist nun der weitere Messkörper 17 das Messgerät 20 und die Verstellvorrichtung 21 auf, kann der Druck im weiteren Messkörper 17 solange erhöht werden, bis die Volumenanzeige im Messgerät 3" der zweiten Messkammer 2" der Volumenanzeige im Messgerät 3' entspricht. Jetzt kann der Druck am Messgerät 20 des weiteren Messkörpers 17 abgelesen werden, welcher Wert dem Innendruck des Prüfkörpers 1 entspricht.

Wesentlich für das Messen des Augeninnendruckes ist, dass die Grösse der Volumenverdrängungen in den Messkammern 2', 2" nach oben begrenzt ist, was durch die Höhe der Standrohre 3' und 3" erreicht wird. Die an den Messkammern 2' und 2" vorgenommenen Verformungen sind daher begrenzt und eine Überlastung des Auges ausgeschlossen.

Zweckmässigerweise wird zuerst die flexible Wandpartie 6' des Messkörpers 2 gegen die Wandpartie 4 des Prüfkörpers 1 gedrückt, bis die Volumenverdrängung in der Messkammer 2' so gross ist, dass die darin befindliche Flüssigkeit im Standrohr 3' sichtbar wird. Alsdann wird der weitere Messkörper 17 mit der flexiblen Wandpartie 19 gegen die flexible Wandpartie 6" der Messkammer 2" gedrückt, bis die darin auftretende Flüssigkeitsvolumenverdrängung im Standrohr 3" sichtbar wird. Mit dem weiteren Messkörper 17 wird nun so stark gedrückt, bis die Flüssigkeitssäulen in den Standrohren 3' und 3" ein bestimmtes Niveau (Marken "a" und "b") erreichen. Will man die Messung bei gleichem Niveaustand vornehmen, können für eine visuelle Einstellung die Marken "a" und "b" entfallen. Die im weiteren Messkörper 17 auftretende Volumenverdrängung wird durch das Messgerät 20 signalisiert, dessen Anzeige einer Masseinheit für den Druck enspricht. Dabei ist die Verformungsarbeit an Prüfkörper 1 gleich jener an der Messkammer 2' und jene an der Messkammer 2" ist gleich jener am weiteren Prüfkörper 17. Ist an der Anzeige des Messgerätes 20 das Verhältnis der an den Messkammern 2' und 2" erfolgten Volumenverdrängungen (Formänderungen) berücksichtigt, signalisiert dieses unmittelbar den im Prüfkörper 1 herrschenden Druck.

Wird die Vorrichtung nach Fig. 3 für die Messung des Innendruckes im Auge verwendet, kann der weitere Messkörper 17, wenn das Messgerät 20 und die Verstellvorrichtung 21 weggelassen werden, mit einem Innendruck von 26,66 mbar versehen werden. Zeigt dann das Messgerät 3' der Messkammer 2' eine grössere Volumenverdrängung an als das Messgerät 3", so bedeutet dies, dass der Innendruck des Auges über dem Normalwert liegt. Ist nun der weitere Messkörper 17 mit Messgerät 20 und Verstellvorrichtung 21 ausgerüstet, kann mit der Verstellvorrichtung 21 der Innendruck des weiteren Messkörpers 17 soweit gesteigert werden, bid die Volumenverdrängungsanzeige im Messgerät 3" der zweiten Messkammer 2" gleich der Volumenverdrändungsanzeige des Messgerätes 3" in der Messkammer 2' ist. Das Messgerät 20 zeigt dann den tatsächlichen Innendruck des Auges an.

In Fig. 4 ist eine weitere Vorrichtung zum Messen des Innendruckes in einem Prüfkörper 1 dargestellt, bei der ein Messkörper 26 verschiebbar in einem Gehäuse 27 gelagert und unter der Wirkung einer Feder 28 steht. Ein Taster 31 stützt sich unter der Wirkung einer weiteren Feder 32 an einer flexiblen Wandpartie 30 des Messkörpers 26 auf, der eine weitere flexible Wandpartie 29 aufweist, die gegen einen Prüfkörper 1 gedrückt wird. Drückt auf die Wandpartie 29 eine Kraft, wird die Feder 28 komprimiert. Die Feder 32 erzeugt die Gegenkraft und wird dabei ebenfalls zusammengedrückt. Die Federn 28 und 32 stellen somit einen ersten Detektor dar, da wenn zusätzliche Kräfte auf die die Tastflächen bildenden Wandpartien 29, 30 wirken, deren Längendifferenz ein Mass für das Verhältnis der an den Wandpartien 29 und 30 erfolgten Verformungen ist. Bei einer Verschiebung des Messkörpers 26 gegenüber dem Gehäuse 27 wird an beiden Wandpartien 29 und 30 die gleiche Verformungsarbeit geleistet und die durch den Taster 31 abgegriffene Deformation der Wandpartie 30, die der Längendifferenz der Kompression der beiden Federn 28 und 32 entspricht, wird in einer Messvorrichtung 33 angezeigt, wobei die Anzeige dem Innendruck im Prüfkörper entspricht. In Fig. 4 sind auch noch zwei weitere Messvorrichtungen dargestellt, wobei a) die getrennte Messung mit Zeigergeräten 34', 34" und b) die Messung mit einem Zeigergerät 34 zeigt bei dem die Nadel beim vorgegebenen Anpressdruck fixiert wird.

Beim Ausführungsbeispiel nach Fig. 5 sind die im Beispiel nach Fig. 3 eine hydropneumatische Feder bildenden Teile durch eine mechanische Feder 35 ersetzt. Diese ist durch die kalottenförmigen, randseits am Gehäuse 11 befestigten Gummikissen 6', 6" auf Druck vorgespannt. Die Feder 35 ist mit einem Zeiger 36 verbunden, der im Gehäuse 11 schwenkbar gelagert ist, und dessen Anzeigenadel mit einer gehäusefesten Marke "b" zusammen wirkt. Ist die Feder 35 durch die axial auf sie wirkenden Kräfte im Gleichgewicht gehalten, weist der Zeiger 36 auf die Marke "b". Ein zweiter Fühler "a" veranlasst ein optisches oder akustisches Warnsignal, wenn im Gleichgewichtsfall die auf die Feder 35 wirkenden Kräfte zu gross werden. Die Messung des Innendruckes eines Prüfkörpers 1 erfolgt in gleicher Weise wie mit der Vorrichtung nach Fig. 3. Zuerst wird die flexible Wandpartie gegen den Prüfkörper 1 gedrückt, ohne die durch den Fühler "a" begrenzte Kraft zu überschreiten. Dabei lenkt der Zeiger 36 nach links aus. Alsdann wird der weitere Messkörper 17 mit der flexiblen Wandpartie 19 gegen das Gummikissen 6" gedrückt, bis der Zeiger 36 erneut auf die Marke "b" weist. In dieser Gleichgewichtslage kann am Messgerät 20 der im Prüfkörper 1 herrschende Druck abgelesen werden.

Anstelle der Gummikissen 6', 6" könnten als Wandseite auch Platten verwendet werden, die mittels Federn am Gehäuse 11 aufgehängt sind und die zur Vorspannkraft der Feder 35 die Gegenkraft erzeugen, welche das Gleichgewicht bewirkt.

Die beschriebene Vorrichtung ist in ihrem Aufbau einfach und leicht bedienbar. Wird die Vorrichtung als Augendruckmessgerät eingesetzt, besteht der Vorteil darin, dass der Augendruck ohne Anästhesiemittel, ja sogar bei geschlossenem Auge gemessen werden kann und viele Patienten dem Augenarzt zugeführt werden können, bevor ein irreversibler Schaden entstanden ist.

Die beschriebenen Messgeräte sind als einfache Anzeigegeräte dargestellt, jedoch ist es möglich, die Messwerte laufend elektronisch zu erfassen, zu speichern und auszuwerten.

## Patentansprüche

1. Tonometer zur Ermittlung des Augeninnendruckes, dadurch gekennzeichnet, dass es einen ersten und einen zweiten Messkörper (2, 17) aufweist, dass der erste Messkörper (2) mit einem Gehäuse versehen ist, an dem zwei je eine erste bzw. zweite Tastfläche bildende Wandteile (6', 6") beweglich gelagert sind, die der Tastfläche abgewandt eine Rückseite aufweisen, dass die Wandteile (6', 6") je durch eine gegen die Rückseite wirkende Vorspannkraft und eine dieser entgegen gerichtete Gegenkraft beaufschlagt sind, welche Kräfte die Wandteile (6', 6") im Gleichgewicht halten, dass ein erster Detektor (b, 3', 3", 10, 28, 32, 36) am Gehäuse (11, 27) vorhanden ist, welcher, wenn zusätzliche Kräfte auf die Tastflächen wirken, ein bestimmtes Verhältnis der an den Wandteilen (6', 6") auftretenden Verformung signalisiert, dass der zweite Messkörper (17) ein zweites Gehäuse (18) mit einem beweglichen, dritten Wandteil (19) aufweist, das aussenseitig eine dritte Tastfläche und innenseitig eine Rückseitenfläche bildet und das von einer gegen die Rückseitenfläche wirkenden Kraft und einer auf die Tastfläche wirkenden Gegenkraft im Gleichgewicht gehalten ist, und dass der zweite Messkörper (17) einen zweiten Detektor (20, 31) aufweist, der mit einem Signalgeber zusammenwirkt, der eine Eichung in Druckeinheiten aufweist, welche proportional dem erwähnten bestimmten Verhältnis der an den ersten beiden Wandteilen (6', 6") aufgebrachten Verformung ist und der zumindest dann ein Signal erzeugt, wenn an den ersten beiden Wandteilen eine (6', 6") Verformung im erwähnten bestimmten Verhältnis aufgebracht ist.

2. Tonometer nach Anspruch 1, dadurch gekennzeichnet, dass die Vorspannkraft durch eine hydropneumatische oder eine mechanische Feder (2', 2", 3', 3", 6', 6", 10 bis 14 bzw. 6', 6", 35) erzeugt wird.

3. Tonometer nach Anspruch 1, dadurch gekennzeichnet, dass im zweiten Messkörper (17) die auf die Rückenfläche wirkende Kraft durch eine pneumatische, hydropneumatische oder mechanische Feder erzeugt ist.

4. Tonometer nach einem der Ansprüche 1 bis 3, dadurch gekennzeichnet, dass die Gegenkräfte je durch eine am Gehäuse befestigte, gummielastische Membrane (6', 6", 19) oder durch mechanische Federn erzeugt ist.

5. Tonometer nach Anspruch 2, dadurch gekennzeichnet, dass die die Tastflächen aufweisenden Wandteile (6', 6") durch eine kalottenförmig gewölbte, mit den Rändern am Gehäuse befestigte, gummielastische Membrane gebildet sind, welche je eine Flüssigkeitskammer (2', 2") begrenzen, und dass die Flüssigkeitskammern (2', 2') durch ein Luftkissen (im Verbindungsbogen 10) verbunden sind und dass jeder Flüssigkeitskammer (2', 2") je ein Detektor (a, b) zugeordnet ist, welche bei auf die Tastflächen wirkenden äusseren Kräften ein bestimmtes Verhältnis der aus den Flüssigkeitskammern (2', 2") verdrängten Volumina signalisieren.

6. Tonometer nach Anspruch 2, dadurch gekennzeichnet, dass die mechanische Feder (35) eine zwischen den Rückseiten auf Druck axial vorgespannte Schraubenfeder ist, und dass der erste Detektor die relative Lage eines Punktes der Feder (35) mit Bezug auf einen gehäusefesten Punkt (6) detektiert.

7. Tonometer nach Anspruch 1, dadurch gekennzeichnet, dass das bewegliche dritte Wandteil (19) durch eine gummielastische kalottenförmige, mit dem Rand am zweiten Gehäuse (18) befestigte Membrane gebildet wird, welche die Gegenkraft erzeugt und eine Flüssigkeitskammer mit einstellbarem Innendruck und angeschlossenem Manometer (20) begrenzt.

8. Tonometer nach Anspruch 7, dadurch gekennzeichnet, dass der Radius der Membrane in grober Näherung dem Radius eines menschlichen Augapfels entspricht.

9. Tonometer nach Anspruch 1 oder 2, dadurch gekennzeichnet, dass die Tastflächen des ersten Messkörpers (2) unter sich gleich sind.

10. Tonometer nach Anspruch 1, dadurch gekennzeichnet, dass der Verschiebeweg der die Tastfläche bildenden Wandteile (6', 6") begrenzt ist.

## Claims

1. Tonometer for determining the internal pressure of eyes, characterised in that it exhibits a first and a second test body (2, 17), in that the first test body (2) is provided with a housing, on which two wall portions (6', 6") forming respectively a first and a second probe surface are movably mounted, which wall portions exhibit a rear surface remote from the probe surface, in that the wall portions (6', 6" are acted upon in each instance by a prestress force acting against the rear surface and a counter-force directed against the prestress force, which forces maintain the wall portions (6', 6") in equilibrium, in that a first detector (b, 3', 3", 10, 28, 32, 36) is provided on the housing (11, 27), which detector, if additional forces act on the probe surfaces, signals a specified ratio of the deformation occuring at the wall portions (6', 6"), in that the second test body (17) exhibits a second housing (18) with a movable, third wall portion (19), which forms on the outside a third probe surface and on the inside a rear-side surface and which is maintained in equilibrium by a force acting against the rear-side surface and a counter-force acting on the probe surface, and in that the second test body (17) exhibits a second detector (20, 31), which cooperates with a signal generator, which exhibits a calibration in pressure units, which is proportional to the said specified ratio of the deformation applied to the first two wall portions (6', 6") and which generates a signal at least when a deformation in the said specified ratio is applied to the first two wall portions (6', 6").

2. Tonometer according to Claim 1, characterised in that the prestress force is generated by a hydropneumatic or a mechanical spring (2', 2", 3', 3", 6', 6", 10 to 14 or 6', 6", 35).

3. Tonometer according to Claim 1, characterised in that in the second test body (17) the force acting on the rear surface is generated by a pneumatic, hydropheumatic or mechanical spring.

4. Tonometer according to one of Claims 1 to 3, characterised in that the counter-forces are generated in each instance by a resilient membrane (6', 6", 19) secured to the housing or by mechanical springs.

5. Tonometer according to Claim 2, characterised in that the wall portions (6', 6") exhibiting the prove surfaces are formed by a resilient membrane which is arched in a cup shape and which is secured by the edges to the housing, which membranes delimit in each instance a liquid chamber (2', 2"), and in that the liquid chambers (2', 2") are connected by a dashpot (in the connecting bend 10), and in that with each liquid chamber (2', 2") there is associated a respective detector (a, b), which detectors, when external forces act on the probe surfaces, signal a specified ratio of the volumes displaced out of the liquid chambers (2', 2").

6. Tonometer according to Claim 2, characterised in that the mechanical spring (35) is a helical spring which is axially prestressed in compression between the rear sides, and in that the first detector detects the relative position of a point of the spring (35) with reference to a point (6) fixed in relation to the housing.

7. Tonometer according to Claim 1, characterised in that the movable third wall portion (19) is formed by a resilient, cup-shaped membrane, which is secured by the edge to the second housing (18) and which generates the counter-force and delimits a liquid chamber with adjustable internal pressure and a manometer (20) connected thereto.

8. Tonometer according to Claim 7, characterised in that, to a rough approximation, the radius of the membrane corresponds to the radius of a human eyeball.

9. Tonometer according to Claim 1 or 2, characterised in that the probe surfaces of the first test body (2) are equal to one another.

10. Tonometer according to Claim 1, characterised in that the displacement path of the wall portions (6', 6") forming the probe surface is limited.

## Revendication

1. Tonomètre de détermination de la pression interne d'un oeil, caractérisé en ce que:
– il présente un premier et un deuxième corps de mesure (2, 17);
– le premier corps de mesure (2) est muni d'un boîtier, sur lequel sont montés mobiles deux éléments de paroi (6', 6") formant chacun respectivement une première et une deuxième surface de touche, éléments de paroi qui présentent un envers opposé à la surface de touche,
– les parties de paroi (6', 6") sont soumises chacune à une force de précontrainte agissant contre l'envers et à une réaction dirigée en sens inverse de elle-ci, lesquelles forces maintiennent en équilibre les éléments de parois (6', 6"),
– un premier détecteur (b, 3', 3", 10, 28, 32, 36) existe dans le boîtier (11, 27) qui, quand des forces supplémentaires agissent sur les surfaces de touche, signale un rapport déterminé de la déformation se produisant sur les éléments de paroi (6′, 6″),
– le deuxième corps de mesure (17) présente un deuxième boîtier (18) avec un troisième élément de paroi mobile (19), qui forme à l'extérieur, une troisième surface de touche et à l'intérieur une surface arrière et qui est maintenu en équilibre par une force agissant contre la surface arrière et une réaction agissant sur la surface de touche et,
– le deuxième corps de mesure (17) présente un deuxième détecteur (20, 31) qui agit de concert avec un transmetteur de signaux, qui présente un étalonnage en unités de pression, lequel est proportionnel au rapport fixé mentionné de la déformation introduite aux deux premiers éléments de parois (6′, 6″) et qui engendre alors au moins un signal quand une déformation est appliquée aux deux premiers éléments de paroi (6′, 6″) dans le rapport fixé mentionné.

2. Tonomètre selon la revendication 1, caractérisé en ce que la force de précontrainte est créée par un ressort hydropneumatique ou mécanique (2′, 2″, 3′, 3″, 6′, 6″, 10 à 14, respectivement 6′, 6″, 35).

3. Tonomètre selon la revendication 1, caractérisé en ce que, dans un deuxième corps de mesure (17), la force agissant sur la surface arrière est créée par un ressort pneumatique, hydropneumatique ou mécanique.

4. Tonomètre selon une des revendications 1 à 3, caractérisé en ce que, la force de réaction est créée chaque fois par une membrane (6′, 6″, 19) élastique en caoutchouc, fixée au boîtier ou par un ressort mécanique.

5. Tonomètre selon la revendication 2, caractérisé en ce que les éléments de paroi (6′, 6″), présentant les surfaces de touche sont formés d'une membrane élastique en caoutchouc, cintrée en forme de calotte, fixée sur les bords du bâti, qui délimitent une chambre à liquide (2′, 2″), et en ce que les chambres à liquides (2′, 2″) sont reliées par un coussin d'air (dans l'arc de raccordement 10) et en ce que, à chaque chambre à liquide (2′, 2″) est appliqué un détecteur (a, b) qui signalent un rapport déterminé des volumes déplacés hors des chambres à liquides (2′, 2″) par suite des forces extérieures agissant sur les surfaces de touche.

6. Tonomètre selon la revendication 2, caractérisé en ce que le ressort mécanique (35) est un ressort à boudin précontraint axialement par pression entre les parois arrière et en ce que le premier détecteur détecte la position relative d'un point du ressort (35) par rapport à un point fixe du boîtier (6).

7. Tonomètre selon la revendication 1, caractérisé en ce que le troisième élément de paroi mobile (19) est formé d'une membrane élastique en caoutchouc en forme de calotte, fixée sur le bord du deuxième boîtier (18) qui crée la force de réaction et délimite une chambre à liquide avec pression intérieure réglable et manomètre (20) branché.

8. Tonomètre selon la revendication 7, caractérisé en ce que le rayon de la membrane en approximation grossière correspond au rayon d'un globe d'oeil humain.

9. Tonomètre selon les revendications 1 ou 2, caractérisé en ce que les surfaces de touche du premier corps de mesure (2) sont égales entre elles.

10. Tonomètre selon la revendication 1, caractérisé en ce que la course de déplacement des éléments de paroi formant les surfaces de touche (6′, 6″) est limitée.

Fig.1

Fig.2

Fig.3

Fig.4

## Fig.5